(19) Europäisches Patentamt

European Patent Office

Office européen des brevets

(11) **EP 1 155 711 B1**

(12) **EUROPEAN PATENT SPECIFICATION**

(45) Date of publication and mention
of the grant of the patent:
**02.11.2005 Bulletin 2005/44**

(51) Int Cl.⁷: **A61N 1/37**

(21) Application number: **01304299.9**

(22) Date of filing: **15.05.2001**

(54) **Method and apparatus for biventricular stimulation and capture monitoring**

Verfahren und Vorrichtung zur biventrikulären Stimulation und zur Überwachung des Einfanges

Procedé et appareil de stimulation biventriculaire et de surveillance de la capture

(84) Designated Contracting States:
**CH DE FR IE IT LI**

(30) Priority: **15.05.2000 US 204277 P**
**01.05.2001 US 847703**

(43) Date of publication of application:
**21.11.2001 Bulletin 2001/47**

(73) Proprietor: **PACESETTER, INC.**
**Sylmar, CA 91392-9221 (US)**

(72) Inventor: **Kroll, Mark W.**
**Simi Valley, CA 93065 (US)**

(74) Representative: **Rees, David Christopher et al**
**Kilburn & Strode**
**20 Red Lion Street**
**London WC1R 4PJ (GB)**

(56) References cited:
**WO-A-99/29368**   **WO-A-99/30777**
**WO-A-99/55415**   **US-A- 5 720 768**
**US-A- 5 902 325**

**Description**

This invention relates generally to a programmable cardiac stimulation device for automatically monitoring capture following delivery of a pacing pulse. More specifically the present invention relates to a biventricular stimulation device in which cross-chamber stimulation and sensing is performed providing effective, synchronous biventricular pacing and reliable capture detection.

In the normal human heart, the sinus node, generally located near the junction of the superior vena cava and the right atrium, constitutes the primary natural pacemaker initiating rhythmic electrical excitation. The cardiac impulse arising from the sinus node is transmitted to the two atrial chambers which in turn contract, pumping blood from those chambers into the respective ventricular chambers. The excitation impulse is further transmitted to the ventricles through the atrioventricular (A-V) node, and via a conduction system comprising the bundle of His, or common bundle, the right and left bundle branches, and the Purkinje fibers. In response, the ventricles contract, the right ventricle pumping unoxygenated blood through the pulmonary artery to the lungs and the left ventricle pumping oxygenated blood through the aorta and arterial tree throughout the body.

Disruption of this natural pacing and conduction system as a result of aging or disease is often successfully treated by artificial cardiac pacing using an implantable pulse generator, from which rhythmic electrical pulses are applied to the heart at a desired rate. One or more heart chambers may be electrically paced depending on the location and severity of the conduction disorder. Recent clinical evidence is revealing that patients suffering from cardiac diseases which affect the contractility of the heart muscle tissue rather than the conduction pathways, generally known as congestive heart failure, can also benefit from cardiac pacing. In such patients, pacing in the atria and ventricles effectively resynchronizes heart chamber contractions thereby improving hemodynamic function of the heart.

With a widening scope of treatment applications, cardiac pacing devices, including pacemakers and implantable defibrillators, have become more and more complex adding numerous programmable features allowing physicians to tailor the pacing therapy to specific patient need. However a basic function of the pacing device, to deliver a pacing pulse of sufficient energy to depolarize the cardiac tissue causing a contraction, a condition commonly known as "capture," and monitoring the subsequent cardiac activity to verify that capture has indeed occurred, continues to be a strong focus of development efforts by pacemaker manufacturers. While it easy to ensure capture by delivering a fixed high-energy pacing pulse as early pacemakers did, this approach quickly depletes battery energy and can result in patient discomfort due to extraneous stimulation of surrounding skeletal muscle tissue.

A common practice in the art has been to deliver pacing pulses at, or slightly higher than the pacing threshold, that is the lowest pacing pulse energy at which capture occurs, in an effort to provide comfortable and effective cardiac pacing without unnecessarily depleting battery energy. Pacing threshold, however, is extremely variable from patient-to-patient due to variations in electrode systems used, electrode positioning, physiological and anatomical variations of the heart itself and so on. Therefore, at the time of device implant, the pacing threshold is determined by the physician who observes an ECG recording while pulse energy is decreased, either by decrementing the pulse amplitude or the pulse width, until "capture" disappears. The pacing pulse energy is then programmed to a setting equal to the lowest pulse energy at which capture still occurred plus some safety margin to allow for small fluctuations in threshold. Selection of this safety margin, however, can be arbitrary. Too low of a safety margin may result in loss of capture, a potentially fatal result for the patient. Too high of a safety margin will lead to premature battery depletion and potential patient discomfort. Furthermore, pacing threshold will vary over time within a patient due to fibrotic encapsulation or the electrode that occurs during the first few weeks after surgery, fluctuations that may occur over the course of a day, fluctuations that occur with changes in medical therapy or changes in disease state and so on.

To address this pressing problem, manufacturers have developed pacemakers that are capable of determining a patient's pacing threshold and automatically adjusting the stimulation pulses to a level just above that which is needed to maintain capture. This approach, called "automatic capture", improves the patient's comfort, reduces the necessity of unscheduled visits to the medical practitioner, and greatly increases the pacemaker's battery life by conserving the energy used to generate stimulation pulses.

However, many of these pacemakers require additional circuitry and/or special sensors that must be dedicated to capture verification. Examples of physiological sensor signals employed for monitoring capture include: thoracic impedance changes that occur as a function of blood volume in the heart; arterial or intra-cardiac pressure changes resulting directly from heart contraction; and blood flow velocity changes associated with ejection of blood from the heart. Several problems arise, however, in attempting to measure physiological signals associated with heart contraction. Measured signals are adversely affected by myoelectric noise, electromagnetic interference, sensor sensitivity, and thoracic changes in pressure or impedance associated with respiration. Furthermore, additional implanted hardware and circuitry are required increasing the complexity of the pacemaker and reducing the precious space available within a pacemaker's casing, and increasing the pacemaker's cost. As a result, manufacturers have attempted to develop automatic capture verification techniques that may be implemented in a typical programmable pacemaker without requiring additional circuitry or special dedicated sensors.

For example, direct measurement of cardiac impedance can be made through monitoring the impedance between two or more cardiac electrodes already connected to the pacing device for cardiac pacing and sensing. Cardiac impedance measurement is performed by delivering an excitation current pulse between two "source" electrodes. The current will be conducted through the cardiac tissue between the two source electrodes producing a voltage differential between two "recording" electrodes. This voltage differential is a function of the total impedance encountered by the "source" electrodes, including the impedance of the electrode-tissue interface and the surrounding tissue and body fluids, and can be given by the equation, $V = I/Z$, where V is the measured voltage differential, I is the applied current pulse, and Z is the total impedance.

During heart contraction, the myocardial walls shorten and thicken and blood is ejected from the ventricles. These changes, particularly the change in blood volume, will have a marked effect on measured impedance. Hence cardiac impedance measurements can be employed in capture detection techniques since cardiac impedance changes are a direct consequence of myocardial contraction.

One cardiac impedance measurement technique is described in U.S. Patent Number 5,902,325 to Condie et al., where an excitation pulse is applied between a bipolar lead ring electrode and the pacemaker housing and impedance is measured between either the same electrode pair or the bipolar lead tip electrode and the pacemaker housing. Such a method could possibly produce an impedance signal containing information regarding changes in thoracic impedance associated with respiration and cardiac impedance associated with heart activity. Filtering of the obtained signal could provide the desired signal components related specifically to cardiac activity and could be used for capture verification.

Another technique used to determine whether capture has occurred that does not require additional circuitry or implanted sensors is monitoring the electrical signals received from the cardiac pacing and sensing electrodes and searching for the presence of an "evoked response" following a pacing pulse. The "evoked response" is the depolarization of the heart tissue in response to a stimulation pulse, in contrast to the "intrinsic response" which is the depolarization of the heart tissue in response to the heart's natural pacing function. Heart activity is typically monitored by the pacemaker by keeping track of the stimulation pulses delivered to the heart and examining, through the leads connected to the heart, electrical signals that are manifest concurrent with depolarization or contraction of muscle tissue (myocardial tissue) of the heart. The contraction of atrial muscle tissue is evidenced by generation of a P-wave, while the contraction of ventricular muscle tissue is evidenced by generation of an R-wave (sometimes referred to as the "QRS" complex).

When capture occurs, the evoked response is an intracardiac P-wave or R-wave that indicates contraction of the respective cardiac tissue in response to the applied stimulation pulse. For example, using such an evoked response technique, if a stimulation pulse is applied to the ventricle (hereinafter referred to as a Vpace), any response sensed by ventricular sensing circuits of the pacemaker immediately following application of the Vpace is presumed to be an evoked response that evidences capture of the ventricles.

However, it would be difficult to detect a true evoked response for several reasons. First, because the evoked response may be obscured by a high energy pacing pulse and therefore difficult to detect and identify. Second, the evoked response may be difficult to distinguish from an intrinsic response since an intrinsic response may occur approximately the same time as an evoked response is expected to occur. Third, the signal sensed by the pacemaker's sensing circuitry immediately following the application of a stimulation pulse may be not be a QRS complex but noise, such as either electrical noise caused, for example, by electromagnetic interference, myoelectric noise caused by skeletal muscle contraction, or "cross talk," defined as signals associated with pacing pulses or intrinsic events occurring in other heart chambers.

Another signal that interferes with the detection of an evoked response, and potentially the most difficult for which to compensate because it is usually present in varying degrees, is lead polarization. A lead/tissue interface is that point at which an electrode of the pacemaker lead contacts the cardiac tissue. Lead polarization is commonly caused by electrochemical reactions that occur at the lead/tissue interface due to application of an electrical stimulation pulse, such as an A-pulse, across the interface. However, because the evoked response is sensed through the same lead electrodes through which the stimulation pulses are delivered, the resulting polarization signal, also referred to herein as an "afterpotential", formed at the electrode can corrupt the evoked response that is sensed by the sensing circuits. This undesirable situation occurs often because the polarization signal can be three or more orders of magnitude greater than the evoked response. Furthermore, the lead polarization signal is not easily characterized; it is a complex function of the lead materials, lead geometry, tissue impedance, stimulation energy and other variables, many of which are continually changing over time.

In each of the above cases, the result may be a false positive detection of an evoked response. Such an error leads to a false capture indication, which in turn leads to missed heartbeats, a highly undesirable and potentially life-threatening situation. Another problem results from a failure by the pacemaker to detect an evoked response that has actually occurred. In that case, a loss of capture is indicated when capture is in fact present, which is also an undesirable situation that will cause the pacemaker to unnecessarily invoke the threshold testing function in a chamber of the heart.

**EP 1 155 711 B1**

Automatic threshold testing is invoked by the pacemaker when loss of atrial or ventricular capture is detected. An automatic pacing energy determination procedure is performed as follows. When loss of capture is detected, the pacemaker increases the pacing pulse output level to a relatively high predetermined testing level at which capture is certain to occur, and thereafter decrements the output level until capture is lost. The pacing energy is then set to a level slightly above the lowest output level at which capture was attained. Thus, capture verification is of utmost importance in proper determination of the pacing threshold.

A further difficulty in achieving optimal sensing of signals of interest, while at the same time delivering efficient pacing, is selecting the most appropriate electrode polarity configuration for pacing and sensing. Historically, either a unipolar or a bipolar configuration is used for pacing and sensing in the heart chambers. In a unipolar configuration, one electrode is positioned at, or near the distal end of the lead body, in contact with the heart tissue. A ground or "indifferent" electrode, commonly the pacemaker housing or "can", is placed some distance away. In bipolar configurations, two electrodes are placed in close proximity to each other at the distal end of the lead body, typically in a "tip" and "ring" configuration, such that both electrodes have contact with the heart tissue.

Determining the ideal polarity configuration remains enigmatic. Medical practitioners tend to have personal preferences and patient variability may make one configuration more successful than another for a multiplicity of reasons. Generally, bipolar configurations require less pacing energy conserving battery longevity, and are less prone to cross-talk than unipolar configurations. Bipolar pacing is preferred over unipolar pacing when extraneous stimulation of skeletal muscle tissue occurs or device pocket infection occurs.

However, unipolar pacing and sensing also has certain advantages. Compared to bipolar configurations, greater sensitivity is achieved. Sensing in the atrium, for example, may be better achieved by unipolar sensing configurations since P-wave signals are relatively small in amplitude. Polarization effects are lessened in unipolar sensing due to a typically large indifferent electrode placed some distance away from the pacing site, therefore particular tasks such as detecting evoked responses to a stimulation pulses may also be better performed in unipolar systems.

New combinations of electrodes are now available, widening the selection a physician has to choose from in deciding which configuration is the most suitable. In dual chamber pacing, an "A-V cross-chamber" electrode configuration, that is, an electrode configuration in which the stimulation device senses cardiac signals between an atrial tip electrode and a ventricular tip electrode, and stimulates each chamber in a unipolar fashion from the respective electrode to the housing (i.e., typically referred to as the case electrode) is possible. For a more detailed description of cross-chamber systems, refer to U.S. Patent 5,522,855 to Hognelid. When such electrodes are implanted, various electrode sensing configurations are possible, e.g., atrial unipolar (A tip-case); ventricular unipolar (V tip-case); atrial-ventricular cross-chamber (A tip-V tip); ventricular unipolar ring (V ring-to-case) or atrial unipolar ring (A ring-to-case).

In biventricular pacing, one bipolar lead is typically placed in the coronary sinus for pacing and sensing in the left ventricle. Another bipolar lead is positioned in the right ventricle for pacing and sensing in right ventricle. Thus, in biventricular pacing, "cross-chamber" configurations could also be used advantageously for pacing and sensing yet such a combination has not yet been made available heretofore. Since synchronous contraction of the right ventricle (RV) and the left ventricle (LV) is desired, both ventricles can be paced simultaneously in a cross-chamber configuration using the tip electrodes of the two leads. The advantage of such a pacing configuration, as will be set forth in the present invention, is that the ring electrodes of the RV and LV pacing leads are not used for pacing, and are therefore available for sensing without the problem of lead polarization. Thus, sensing can also be performed in a cross-chamber configuration, across the ventricles for detecting and verifying capture.

It would thus be desirable to provide a stimulation device for biventricular stimulation in which the stimulation energy is minimized, and reliable capture verification is performed. It would also be desirable to provide a system for biventricular sensing in which an electrode configuration is used that avoids the negative effect of polarization and noise on sensing and capture verification. It would further be desirable to enable the pacemaker to perform ventricular capture verification without requiring dedicated circuitry and/or special sensors.

The present invention addresses these and other problems by providing a multi-chamber stimulation device for reliably and automatically verifying capture during cardiac pacing. An exemplary use of the present invention is in biventricular pacing systems employing bipolar electrode pairs on leads positioned in the right ventricle and in the coronary sinus.

One feature of the present invention is to provide efficient, synchronous biventricular pacing. Having one bipolar pacing lead positioned for pacing and sensing in the left ventricle and another bipolar pacing lead positioned for pacing and sensing in the right ventricle, a cross-chamber pacing configuration between two electrodes located on the two different leads, preferably the "tip" electrodes, is performed for capturing both the right and left ventricles simultaneously. Electrode polarity can be programmably selected, thus influencing the sequence of activation within the ventricles.

Another feature of the present invention is the choice of stimulation pulse delivered. During cross-chamber pacing, either a biphasic pulse or a balanced monophasic pulse may be delivered. However, the present invention also provides a "biventricular pacing pulse," in which a positive going pulse is delivered to one electrode in one ventricle and an inverted, negative going pulse is delivered to another electrode in the other ventricle. By delivering such a "biventricular pacing pulse," a larger potential difference is established across the ventricles between the two electrodes, improving the recruitment of Purkinje fibers and other conductive elements of the cardiac tissue, thus enhancing conduction of the stimulating pulses through the cardiac tissue.

A further feature of the present invention is to provide reliable capture verification by sensing in either a bipolar or cross-chamber configuration, preferably in a ring-to-ring fashion, using distinctly different sensing electrode pairs than the electrode pairs used for stimulation. In this way problems of lead polarization are avoided making detection of the evoked response for capture verification accurate and reliable. Therefore, another feature of the present invention is a programmably available plurality of electrode combinations for sensing, in which the electrode pair for sensing is different than the electrode pair for pacing. Electrode polarity can be programmably selected thus influencing the directional pathway of sensing within the ventricles.

In another embodiment of the present invention, cardiac impedance measurements are made in order to detect and verify capture. An impedance measurement is made in a cross-chamber arrangement by applying an excitation current pulse between the two ventricular tip electrodes and measuring the resulting voltage differential between the two ring electrodes. Hence a cross-ventricular impedance measurement is made giving a highly sensitive indication of the actual mechanical contraction and ejection of blood from the ventricles, in other words, a very reliable method of capture verification.

In another alternative embodiment of the present invention, one bipolar electrode pair on one ventricular lead is used to pace while the second bipolar electrode pair on the second ventricular lead is used to sense. A suprathreshold pacing pulse delivered by the first bipolar pair will capture the first ventricle and cause a depolarization wave to be conducted to the second ventricle, ultimately capturing both ventricles. The R-wave depolarization detected by the sensing bipolar electrode pair in the second ventricle thus verifies that capture in both ventricles has occurred. If no R-wave depolarization is sensed in the second ventricle, loss of capture in the first ventricle is detected. Such monitoring of ventricular capture can be termed "cross-tracking."

The present invention thus achieves efficient synchronous biventricular pacing using cross-chamber electrode configurations which minimize pacing energy requirements, and reliable capture detection by using cross-chamber electrode configurations which avoid problems of lead polarization.

The above and further features, advantages and benefits of the present invention will be apparent upon consideration of the present description taken in conjunction with the accompanying drawings, in which:

FIG. 1 is a simplified, partly cutaway view illustrating an implantable stimulation device in electrical communication with at least three leads implanted into a patient's heart for delivering multi-chamber stimulation and shock therapy;

FIG. 2 is a functional block diagram of the multi-chamber implantable stimulation device of FIG. 1, illustrating the basic elements that provide cardioversion, defibrillation and pacing stimulation in four chambers of the heart;

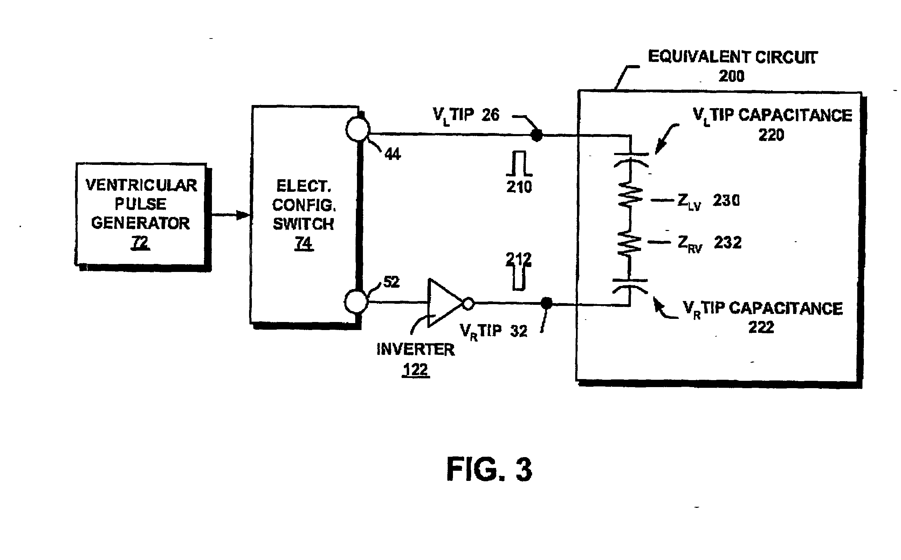

FIG. 3 is a block diagram of the stimulation device of FIGS. 1 and 2, illustrating a switch bank with three connection ports;

FIG. 4 is a circuit diagram illustrating the biventricular pacing electrode configuration used by the stimulation device of FIG. 2 according to one embodiment of the present invention;

FIG. 5 is an illustration of the biventricular sensing electrode configuration used by the stimulation device of FIG. 2 according to one embodiment of the present invention;

FIG. 6 is an illustration of an alternative biventricular sensing electrode configuration used by the stimulation device of FIG. 2;

FIG. 7 is a graphical depiction of the electromyographic signal received by the biventricular sensing electrode configuration of FIG. 5 when loss of capture has occurred;

FIG. 8 is a graphical depiction of the electromyographic signal received by the biventricular sensing electrode configuration of FIG. 5 when biventricular capture has occurred;

FIG. 9 is a circuit diagram illustrating an impedance measurement configuration according to an alternative embodiment of the present invention; and

FIG. 10 is a graphical depiction of the impedance signal measured by the impedance measurement configuration of FIG. 9.

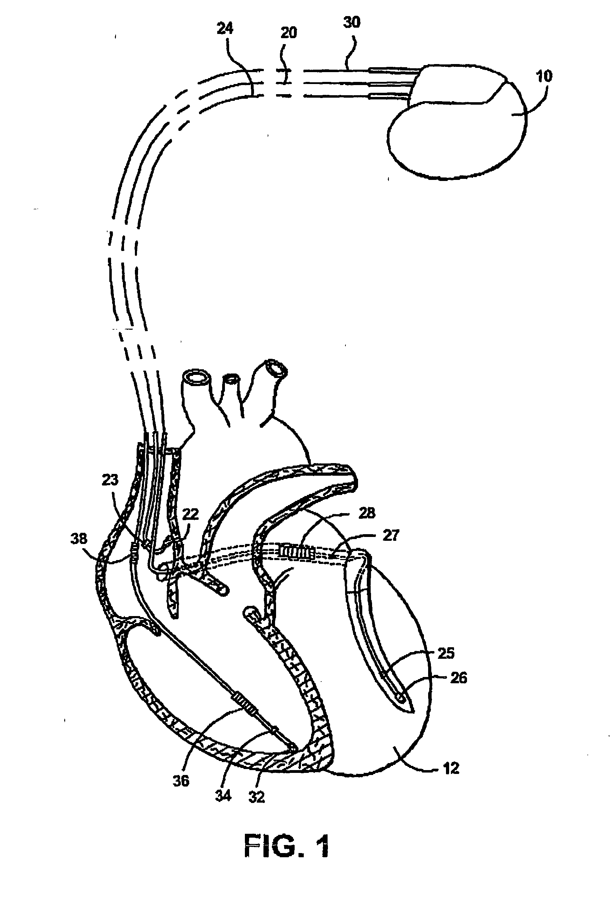

FIG. 1 illustrates a stimulation device 10 in electrical communication with a patient's heart 12 by way of three leads 20, 24 and 30 suitable for delivering multi-chamber stimulation and shock therapy. To sense atrial cardiac signals and to provide right atrial chamber stimulation therapy, the stimulation device 10 is coupied to an implantable right atrial lead 20 having at least an atrial tip electrode 22, which typically is implanted in the patient's right atrial appendage.

To sense left atrial and ventricular cardiac signals and to provide left-chamber pacing therapy, the stimulation device 10 is coupled to a "coronary sinus" lead 24 designed for placement in the "coronary sinus region" via the coronary sinus so as to place a distal electrode adjacent to the left ventricle and additional electrode(s) adjacent to the left atrium.

As used herein, the phrase "coronary sinus region" refers to the vasculature of the left ventricle, including any portion of the coronary sinus, great cardiac vein, left marginal vein, left posterior ventricular vein, middle cardiac vein, and/or small cardiac vein or any other cardiac vein accessible by the coronary sinus.

Accordingly, the coronary sinus lead 24 is designed to receive atrial and ventricular cardiac signals and to deliver: left ventricular pacing therapy using at least a left ventricular tip electrode 26, left atrial pacing therapy using at least a left atrial ring electrode 27, and shocking therapy using at least a left atrial coil electrode 28. For a more detailed description of a coronary sinus lead, reference is made to U.S. Patent Application No. 09/457,277, titled "A Self-Anchoring Steerable Coronary Sinus Lead" (Pianca et. al), and U.S. Patent No. 5,466,254, titled "Coronary Sinus Lead with Atrial Sensing Capability" (Helland).

The stimulation device 10 is also shown in electrical communication with the patient's heart 12 by way of an implantable right ventricular lead 30 having, in this embodiment, a right ventricular tip electrode 32, a right ventricular ring electrode 34, a right ventricular (RV) coil electrode 36, and an SVC coil electrode 38. Typically, the right ventricular lead 30 is transvenously inserted into the heart 12 so as to place the right ventricular tip electrode 32 in the right ventricular apex so that the RV coil electrode 36 will be positioned in the right ventricle and the SVC coil electrode 38 will be positioned in the superior vena cava. Accordingly, the right ventricular lead 30 is capable of receiving cardiac signals, and delivering stimulation in the form of pacing and shock therapy to the right ventricle. While a preferred embodiment of the present invention is intended for use in a biventricular pacing and sensing mode employing coronary sinus lead 24 and right ventricular lead 30, the teaching of the present invention are not limited to this specific implementation.

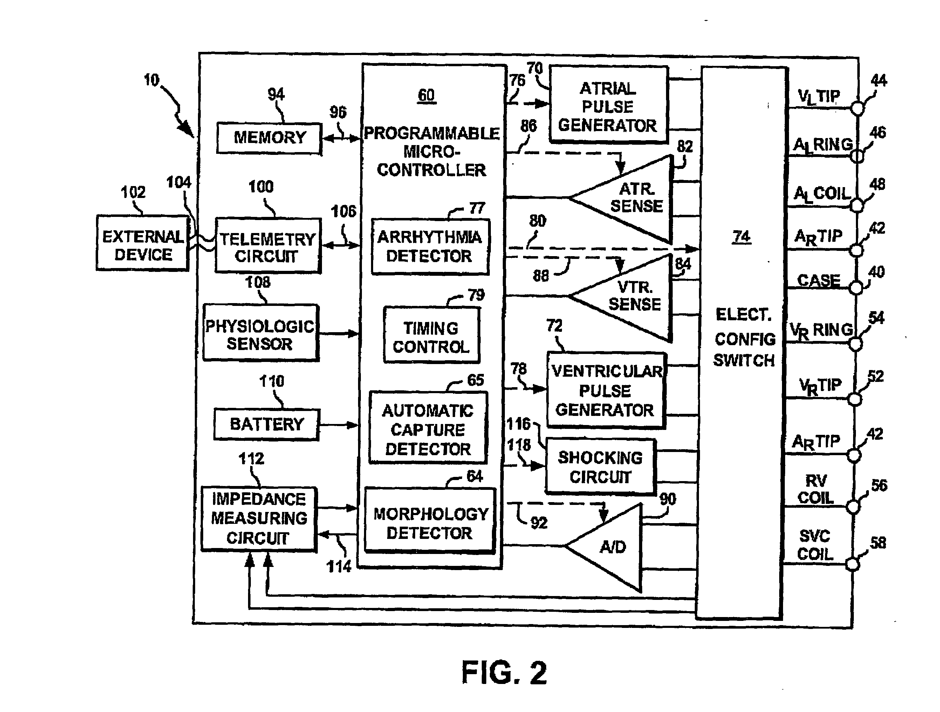

**FIG. 2** illustrates a simplified block diagram of the multi-chamber implantable stimulation device 10, which is capable of treating both fast and slow arrhythmias with stimulation therapy, including cardioversion, defibrillation, and pacing stimulation. While a particular multi-chamber device is shown, this is for illustration purposes only, and one of skill in the art could readily duplicate, eliminate or disable the appropriate circuitry in any desired combination to provide a device capable of treating the appropriate chamber(s) with cardioversion, defibrillation and/or pacing stimulation.

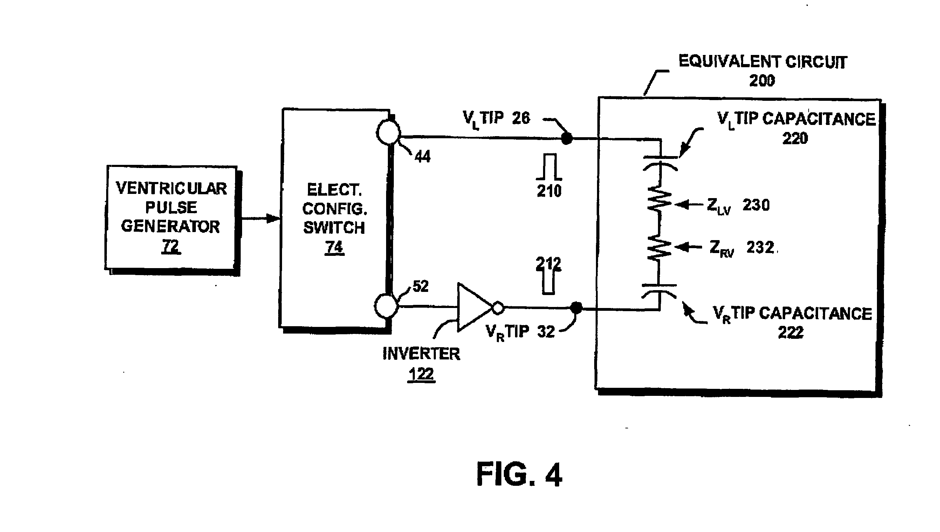

The stimulation device 10 includes a housing 40 which is often referred to as "can", "case" or "case electrode", and which may be programmably selected to act as the return electrode for all "unipolar" modes. The housing 40 may further be used as a return electrode alone or in combination with one or more of the coil electrodes 28, 36, or 38, for shocking purposes. The housing 40 further includes a connector (or a plurality of connectors 150, 152, 154, as it will be described in connection with **FIG. 4**) having a plurality of terminals, 42, 43, 44, 45, 46, 48, 52, 54, 56, and 58. For convenience, the names of the electrodes to which they are connected are shown next to the corresponding terminals. As an example, to achieve right atrial sensing and pacing, the connector includes at least a right atrial tip terminal 42 adapted for connection to the atrial ($A_R$) tip electrode 22.

To achieve left chamber sensing, pacing and/or shocking, the connector includes at least a left ventricular ($V_L$) tip terminal 44, a left ventricular ($V_L$) ring terminal 45, and a left atrial ($A_L$) ring terminal 46, and a left atrial ($A_L$) shocking terminal (coil) 48, which are adapted for connection to the left ventricular tip electrode 26, the left atrial tip electrode 27, and the left atrial coil electrode 28, respectively.

To support right chamber sensing, pacing and/or shocking, the connector further includes a right ventricular ($V_R$) tip terminal 52, a right ventricular ($V_R$) ring terminal 54, a right ventricular (RV) shocking terminal (coil) 56, and an SVC shocking terminal (coil) 58, which are adapted for connection to the right ventricular tip electrode 32, right ventricular ring electrode 34, the RV coil electrode 36, and the SVC coil electrode 38, respectively.

In the embodiment of **FIGS. 1** and **2**, and as further illustrated in **FIG. 4**, the stimulation device 10 is illustrated to include three bipolar connection ports 150, 152, 154. A left ventricular/atrial connection port (LV / LA connection port) 150 accommodates the left ventricular lead (LV lead) 24 with terminals 44, 45, 46, 48 that are associated with the left ventricular tip electrode (LV tip electrode) 26, the left ventricular ring electrode (LV ring electrode) 25, the left atrial ring electrode (LA ring electrode) 27, and the left atrial coil electrode (LA coil electrode) 28, respectively.

A right ventricular connection port (RV connection port) 152 accommodates the right ventricular lead (RV lead) 30 with terminals 52, 54, 56, 58 that are associated with the right ventricular tip electrode (RV tip electrode) 32, the right ventricular ring electrode (RV ring electrode) 34, the right ventricular coil electrode (RVCE) 36, and the right ventricular SVC coil electrode (RV SVC coil electrode) 38, respectively.

A right atrial connection port (RA connection port) 154 accommodates the right atrial lead (RA lead) 20 with terminals that are associated with the right atrial tip electrode (RA tip electrode) 22 and the right atrial ring electrode (RA ring electrode) 23, respectively.

It is recognized that numerous variations exist in which combinations of unipolar, bipolar and/or multipolar leads may be positioned at desired locations within the heart in order to provide multichamber or multisite stimulation.

At the core of the stimulation device 10 is a programmable microcontroller 60 that controls the various modes of stimulation therapy. As is well known in the art, the microcontroller 60 typically includes a microprocessor, or equivalent control circuitry, designed specifically for controlling the delivery of stimulation therapy, and may further include RAM or ROM memory, logic and timing circuitry, state machine circuitry, and I/O circuitry. Typically, the microcontroller 60 includes the ability to process or monitor input signals (data) as controlled by a program code stored in a designated block of memory. The details of the design and operation of the microcontroller 60 are not critical to the present invention. Rather, any suitable microcontroller 60 may be used that carries out the functions described herein. The use of microprocessor-based control circuits for performing timing and data analysis functions are well known in the art.

Representative types of control circuitry that may be used with the present invention include the microprocessor-based control system of U.S. Patent No. 4,940,052 (Mann et. al), and the state-machine of U.S. Patent Nos. 4,712,555 (Sholder) and 4,944,298 (Sholder). For a more detailed description of the various timing intervals used within the stimulation device and their inter-relationship, refer to U.S. Patent 4,788,980 (Mann et. al). These patents (Nos. 4,940,052; 4,712,555; 4,944,298; and 4,788,980) are incorporated herein by reference.

As shown in **FIG. 2**, an atrial pulse generator 70 and a ventricular pulse generator 72 generate pacing stimulation pulses for delivery by the right atrial lead 20, the right ventricular lead 30, and/or the coronary sinus lead 24 via a switch bank 74. It is understood that in order to provide stimulation therapy in each of the four chambers of the heart, the atrial pulse generator 70 and the ventricular pulse generator 72 may include dedicated, independent pulse generators, multiplexed pulse generators, or shared pulse generators. The atrial pulse generator 70 and the ventricular pulse generator 72 are controlled by the microcontroller 60 via appropriate control signals 76 and 78, respectively, to trigger or inhibit the stimulation pulses.

The microcontroller 60 further includes timing control circuitry 79 which is used to control the timing of such stimulation pulses (e.g. pacing rate, atrio-ventricular (AV) delay, atrial interconduction (A-A) delay, or ventricular interconduction (V-V) delay, etc.), as well as to keep track of the timing of refractory periods, PVARP intervals, noise detection windows, evoked response windows, alert intervals, marker channel timing, etc.

In one embodiment of the present invention, the switch bank 74 includes a plurality of switches for connecting the desired electrodes to the appropriate I/O circuits, thereby providing complete electrode programmability. Accordingly, the switch bank 74, in response to a control signal 80 from the microcontroller 60, determines the polarity of the stimulation pulses (e.g. unipolar, bipolar, cross-chamber, etc.) by selectively closing the appropriate combination of switches (not shown) as is known in the art

Atrial sensing circuits 82 and ventricular sensing circuits 84 may also be selectively coupled to the right atrial lead 20, coronary sinus lead 24, and the right ventricular lead 30, through the switch bank 74, for detecting the presence of cardiac activity in each of the four chambers of the heart. Accordingly, the atrial and ventricular sensing circuits 82 and 84 may include dedicated sense amplifiers, multiplexed amplifiers, or shared amplifiers.

The ventricular sensing circuits 84 are coupled to the coronary sinus lead 24 and the right ventricular lead 30 through switch bank 74 for detecting the presence of cardiac activity within the right and/or left ventricles. The switch bank 74 determines the "sensing polarity" of the cardiac signal by selectively closing the appropriate switches. In this way, the clinician may program the sensing polarity independent of the stimulation polarity. The electrode programmability includes selection of the pacing polar configuration, selection of the sensing polar configuration as well as designation of the positive, negative or indifferent poles for each polar configuration.

Each of the atrial sensing circuit 82 or the ventricular sensing circuit 84 preferably employs one or more low power, precision amplifiers, each with programmable gain and/or automatic gain control, bandpass filtering, and a threshold detection circuit, to selectively sense the cardiac signal of interest. The automatic gain control enables the stimulation device 10 to deal effectively with the difficult problem of sensing the low amplitude signal characteristics of atrial or ventricular fibrillation.

The outputs of the atrial and ventricular sensing circuits 82 and 84 are connected to the microcontroller 60 for triggering or inhibiting the atrial and ventricular pulse generators 70 and 72, respectively, in a demand fashion, in response to the absence or presence of cardiac activity, respectively, in the appropriate chambers of the heart. The atrial and ventricular sensing circuits 82 and 84, in turn, receive control signals over signal lines 86 and 88 from the microcontroller 60, for controlling the gain, threshold, polarization charge removal circuitry (not shown), and the timing of any blocking circuitry (not shown) coupled to the inputs of the atrial and ventricular sensing circuits 82 and 84.

For arrhythmia detection, the stimulation device 10 includes an arrhythmia detector 77 (**FIG. 2**) that utilizes the atrial and ventricular sensing circuits 82 and 84 to sense cardiac signals, for determining whether a rhythm is physiologic or pathologic. As used herein "sensing" is reserved for the noting of an electrical signal, and "detection" is the processing of these sensed signals and noting the presence of an arrhythmia. The timing intervals between sensed events (e.g. P-waves, R-waves, and depolarization signals associated with fibrillation which are sometimes referred to as "F-waves" or "Fib-waves") are then classified by the microcontroller 60 by comparing them to a predefined rate zone limit (e.g. bradycardia, normal, low rate VT, high rate VT, and fibrillation rate zones) and various other characteristics (e.g. sudden onset, stability, physiologic sensors, and morphology, etc.) in order to determine the type of remedial therapy that is needed (e.g. bradycardia pacing, anti-tachycardia pacing, cardioversion shocks or defibrillation shocks, collectively referred to as "tiered therapy").

Cardiac signals are also applied to the inputs of an analog-to-digital (A/D) data acquisition system 90. The data acquisition system 90 is configured to acquire intracardiac electrogram signals, convert the raw analog data into digital signals, and store the digital signals for later processing and/or telemetric transmission to an external device 102. The data acquisition system 90 is coupled to the right atrial lead 20, the coronary sinus lead 24, and the right ventricular lead 30 through the switch bank 74 to sample cardiac signals across any pair of desired electrodes.

Advantageously, the data acquisition system 90 may be coupled to the microcontroller 60 or another detection circuitry, for detecting an evoked response from the heart 12 in response to an applied stimulus, thereby aiding in the detection of "capture". Capture occurs when an electrical stimulus applied to the heart is of sufficient energy to depolarize the cardiac tissue, thereby causing the heart muscle to contract. The microcontroller 60 detects a depolarization signal during a window following a stimulation pulse, the presence of which indicates that capture has occurred. The microcontroller 60 includes an automatic capture detector 65 that enables capture detection by triggering the ventricular pulse generator 72 to generate a stimulation pulse, starting a capture detection window using the timing circuitry within the microcontroller 60, and enabling the data acquisition system 90 via control signal 92 to sample the cardiac signal that falls in the capture detection window and, based on the amplitude of the sampled cardiac signal, determines if capture has occurred.

The microcontroller 60 is further coupled to a memory 94 by a suitable data/address bus 96, wherein the programmable operating parameters used by the microcontroller 60 are stored and modified, as required, in order to customize the operation of the stimulation device 10 to suit the needs of a particular patient. Such operating parameters define, for example, pacing pulse amplitude, pulse duration, electrode polarity, rate, sensitivity, automatic features, arrhythmia detection criteria, and the amplitude, waveshape and vector of each shocking pulse to be delivered to the patient's heart 12 within each respective tier of therapy. A feature of the stimulation device 10 is the ability to sense and store a relatively large amount of data (e.g. from the data acquisition system 90), which data may then be used for subsequent analysis to guide the programming of the stimulation device 10.

Advantageously, the operating parameters of the stimulation device 10 may be non-invasively programmed into the memory 94 through a telemetry circuit 100 in telemetric communication with the external device 102, such as a programmer, transtelephonic transceiver, or a diagnostic system analyzer. The telemetry circuit 100 is activated by the microcontroller 60 by a control signal 106. The telemetry circuit 100 advantageously allows intracardiac electrograms and status information relating to the operation of the stimulation device 10 (as contained in the microcontroller 60 or memory 94) to be sent to the external device 102 through the established communication link 104.

The stimulation device 10 may further include a physiologic sensor 108, commonly referred to as a "rate-responsive" sensor because it is typically used to adjust pacing stimulation rate according to the exercise state of the patient. However, the physiological sensor 108 may further be used to detect changes in cardiac output, changes in the physiological condition of the heart, or diurnal changes in activity (e.g. detecting sleep and wake states). Accordingly, the microcontroller 60 responds by adjusting the various pacing parameters (such as rate, AV Delay, V-V Delay, etc.) at which the atrial and ventricular pulse generators 70 and 72 generate stimulation pulses.

While the physiologic sensor 108 is shown as being included within the stimulation device 10, it is to be understood that the physiologic sensor 108 may alternatively be external to the stimulation device 10, yet still be implanted within, or carried by the patient. A common type of rate responsive sensor is an activity sensor, such as an accelerometer or a piezoelectric crystal, which is mounted within the housing 40 of the stimulation device 10. Other types of physiologic sensors are also known, for example, sensors which sense the oxygen content of blood, respiration rate and/or minute ventilation, pH of blood, ventricular gradient, etc. However, any sensor may be used which is capable of sensing a physiological parameter which corresponds to the exercise state of the patient.

The stimulation device 10 additionally includes a power source such as a battery 110 that provides operating power to all the circuits shown in **FIG. 2**. For the stimulation device 10, which employs shocking therapy, the battery 110 must be capable of operating at low current drains for long periods of time, and also be capable of providing high-current pulses (for capacitor charging) when the patient requires a shock pulse. The battery 110 must preferably have a predictable discharge characteristic so that elective replacement time can be detected. Accordingly, the stimulation device 10 can employ lithium/silver vanadium oxide batteries.

The stimulation device 10 further includes a magnet detection circuitry (not shown), coupled to the microcontroller 60. The purpose of the magnet detection circuitry is to detect when a magnet is placed over the stimulation device 10, which magnet may be used by a clinician to perform various test functions of the stimulation device 10 and/or to signal the microcontroller 60 that an external programmer 102 is in place to receive or transmit data to the microcontroller 60 through the telemetry circuit 100.

As further shown in **FIG. 2**, the stimulation device 10 is shown as having an impedance measuring circuit 112 which is enabled by the microcontroller 60 by a control signal 114. Certain applications for an impedance measuring circuit 112 include, but are not limited to, lead impedance surveillance during the acute and chronic phases for proper lead positioning or dislodgment; detecting operable electrodes and automatically switching to an operable pair if dislodgment occurs; measuring respiration or minute ventilation; measuring thoracic impedance for determining shock thresholds; measuring stroke volume; and detecting the opening of the valves, etc. The impedance measuring circuit 112 is advantageously coupled to the switch bank 74 so that any desired electrode may be used. In an alternative embodiment of the present invention, an impedance measurement circuit 112 is used for measuring cardiac impedance in a method of verifying capture. Changes in cardiac impedance associated with changes in heart chamber blood volume reflect the filling and ejection of blood from the heart. A cardiac impedance deflection results when the ventricles contract and eject blood can therefore be used in verifying that capture has occurred following a pacing pulse. In this embodiment, an excitation current pulse is applied between left ventricular ($L_V$) tip electrode 26 and the right ventricular ($R_V$) tip electrode 32 and the impedance measurement is made across left atrial ($L_A$) ring electrode 27 and right ventricular ($R_V$) ring electrode 34.

It is a function of the stimulation device 10 to operate as an implantable cardioverter/defibrillator (ICD) device. That is, it must detect the occurrence of an arrhythmia, and automatically apply an appropriate electrical shock therapy to the heart aimed at terminating the detected arrhythmia. To this end, the microcontroller 60 further controls a shocking circuit 116 by way of a control signal 118. The shocking circuit 116 generates shocking pulses of low (up to 0.5 Joules), moderate (0.5 - 10 Joules), or high (11 to 40 Joules) energy, as controlled by the microcontroller 60. Such shocking pulses are applied to the patient's heart through at least two shocking electrodes, and as shown in this embodiment, selected from the left atrial coil electrode 28, the RV coil electrode 36, and/or the SVC coil electrode 38 (**FIG. 1**). As noted above, the housing 40 may act as an active electrode in combination with the RV electrode 36, or as part of a split electrical vector using the SVC coil electrode 38 or the left atrial coil electrode 28 (i.e., using the RV electrode as common electrode).

Cardioversion shocks are generally considered to be of low to moderate energy level (so as to minimize pain felt by the patient), and/or synchronized with an R-wave and/or pertaining to the treatment of tachycardia. Defibrillation shocks are generally of moderate to high energy level (i.e., corresponding to thresholds in the range of 5-40 Joules), delivered asynchronously (since R-waves may be too disorganized), and pertaining exclusively to the treatment of fibrillation. Accordingly, the microcontroller 60 is capable of controlling the synchronous or asynchronous delivery of the shocking pulses.

In this embodiment, a control program executed by microprocessor 60 is comprised of multiple integrated program modules, with each module bearing responsibility for controlling one or more functions of the stimulation device 10. For example, one program module may control the delivery of stimulating pulses to the heart 12, while another may control the verification of ventricular capture and ventricular pacing energy determination. In effect, each program module is a control program dedicated to a specific function or set of functions of the stimulation device 10.

Having described the environment in which the present invention operates, an exemplary preferred embodiment of the present invention will now be described in detail. **FIG. 4** is an illustration depicting an equivalent circuit diagram of the electrode configuration used for biventricular pacing in accordance with a preferred embodiment of the present invention. A cross-chamber electrode configuration, defined as a polarity configuration comprising two or more electrodes existing on at least two different lead bodies located in two different heart chambers, is used for cross-ventricular pacing. A pacing pulse is delivered by the ventricular pulse generator 72 between the left ventricular ($V_L$) tip electrode 26 and the right ventricular ($V_R$) tip electrode 32. To maintain a potential difference between these two tip electrodes 26 and 32, the ventricular pacing pulse is inverted by an inverter 122 prior to delivering the pulse to the right ventricular ($V_R$) tip electrode 32. A positive pacing pulse 210 is delivered to the left ventricular ($V_L$) tip electrode 26 and a negative pacing pulse 212 is delivered to the right ventricular ($V_R$) tip electrode 32. Thus, a cross-chamber, biventricular pacing configuration is provided.

Such a configuration could be referred to as "pseudo unipolar" since stimulation is performed between two electrodes not located on the same lead body and not involving the can 40, but rather in different cardiac chambers, yet each electrode effectively activates its respective chamber. The potential difference between the left ventricular ($V_L$) tip electrode 26 and the right ventricular ($V_R$) tip electrode 32 will result in conduction of the stimulating pulse through the conductive elements of the cardiac tissues, for example the Purkinje fibers, and the intercalated discs of the myocardial cells, causing depolarization in both ventricles, thereby achieving synchronous biventricular pacing.

This conductive pathway is represented by an equivalent circuit 200. Besides the conductive elements of the cardiac tissues mentioned above, the equivalent circuit 200 also includes a capacitive element 220 associated with the left ventricular ($V_L$) tip electrode 26 and a capacitive element 222 associated with the right ventricular ($V_R$) tip electrode 32. In this equivalent circuit 200 the characteristic tissue impedance of the left ventricular tissue is generally represented by a resistive element $Z_{LV}$ 230, and the characteristic tissue impedance of the right ventricular tissue is generally represented by a resistive element $Z_{RV}$ 232.

Since the tip electrodes 26 and 32 are in relatively close proximity to each other and are approximated by excitable cardiac tissue including myocardial cells, the Purkinje fibers, and cardiac bundles, direct conduction of excitation pulses is possible between the two ventricles. Furthermore, since this conductive pathway is not dependent on the right or left bundle branches extending into the left ventricle and the right ventricle from the Bundle of His and the atrioventricular node, ventricular stimulation in this cross-chamber configuration is less interrupted by cardiac conduction disorders such as Bundle Branch Block associated with conduction disease or aging that can affect the heart's normal conduction pathways.

The advantages of this cross-chamber pacing configuration are: 1) the stimulation energy required is expected to be much less than that required for true unipolar pacing, and 2) the left and right ring electrodes 27 and 34 located on the coronary sinus lead 24 and RV lead 30, respectively, are not used for stimulation. Since these two ring electrodes 27 and 34 do not become polarized during a ventricular stimulation pulse (i.e., Vpace) as they would during bipolar stimulation, they are advantageously available for sensing the evoked response immediately following the stimulation pulse as will be described later in detail.

A further aspect of the present invention is the programmable selection of the direction of current flow between the stimulating electrodes 26, 32. In the embodiment shown in **FIG. 4**, the direction of the current flow will be from the right ventricular ($V_R$) tip electrode 34 to the left ventricular ($V_L$) tip electrode 26 since the negative going pulse is applied to the right ventricular ($V_R$) tip electrode 32, and the positive going pulse is applied to the left ventricular ($V_L$) tip electrode 26. By selectively applying the reverse voltage polarity, that is the negative going pulse to the left ventricular ($V_L$) tip electrode 26, and the positive going pulse to the right ventricular ($V_R$) tip electrode 32, the direction of the current flow will be reversed. Hence, the sequence of myocardial tissue activation can be influenced by selecting the direction of current flow. This selection allows specificity of activation sequence based on the patient's need. For example, in a patient suffering from dilated cardiomyopathy, typically the left ventricle is predominately affected in the earlier stages of the disease. The dilated left ventricle has diminished contractility causing its contraction to be slower and weaker than the still healthy right ventricle. Thus, by selecting the stimulation pathway direction from the left ventricle to the right ventricle, the slower left ventricle contraction is initiated prior to the faster right ventricle contraction, yielding superior synchronization of right ventricle and left ventricle contractions.

A further aspect of the present invention is the selection of the pacing pulse morphology. In the embodiment of **FIG. 4**, a biventricular pacing pulse is illustrated where a positive going pulse 210 is applied to one electrode and a negative going pulse 212 is applied to a second electrode. Other pacing pulse morphologies are possible. For example, a balanced monophasic pacing pulse, or a biphasic pacing pulse could be applied to one electrode tip while the other electrode tip remains neutral, functioning as the return path for the conducted current.

Yet a further aspect of the present invention is the enhancement of electrode tip geometry such that capacitive coupling between the points of electrode contact is increased, thereby improving the conductivity directly between the right and left tip electrodes 26 and 32, respectively. Such enhancement is preferably achieved by manufacturing the electrode tip with a rough surface to increase its surface area.

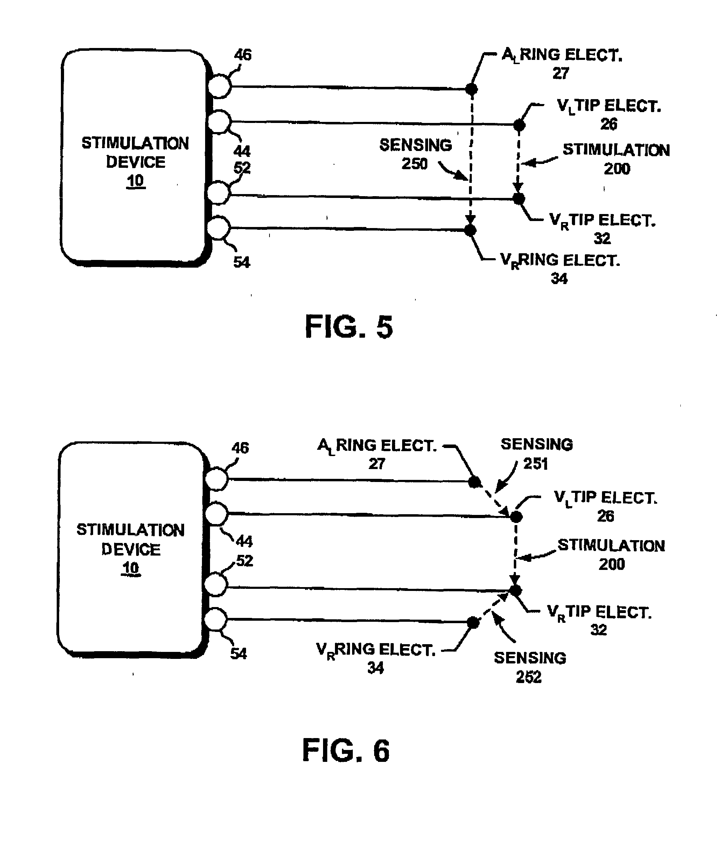

**FIG. 5** depicts an exemplary sensing configuration for a biventricular stimulation system in accordance with the present invention. Ventricular sensing circuitry 84 samples the myoelectric signal between the left atrial ($A_L$) ring electrode 27 and the right ventricular ($V_R$) ring electrode 34. Thus a cross-chamber, cross-ventricular sensing configuration is provided. Ventricular pulse generator 72 (**FIG. 2**) delivers pacing pulses via the left and right tip electrodes 26 and 32, respectively, as described earlier in conjunction with **FIG. 4**. In this way, the cross-chamber sensing configuration is not impaired by lead polarization effects, thus providing a superior sensing configuration for detecting and verifying capture.

The left ventricular ($V_L$) tip electrode 26 and left atrial ($A_L$) ring electrode 27 can be connected to the stimulation device 10 via a single lead body 24 (**FIG. 1**), with bipolar connections to the left ventricular ($V_L$) tip terminal 44 and the atrial ring terminal 46. The right ventricular ($V_R$) tip electrode 32 and the right ventricular ($V_R$) ring electrode 34 are connected to the stimulation device 10 via a single lead body 30 (**FIG. 1**), with bipolar connections to the right ventricular ($V_R$) tip terminal 52 and the right ventricular ($V_R$) ring terminal 54.

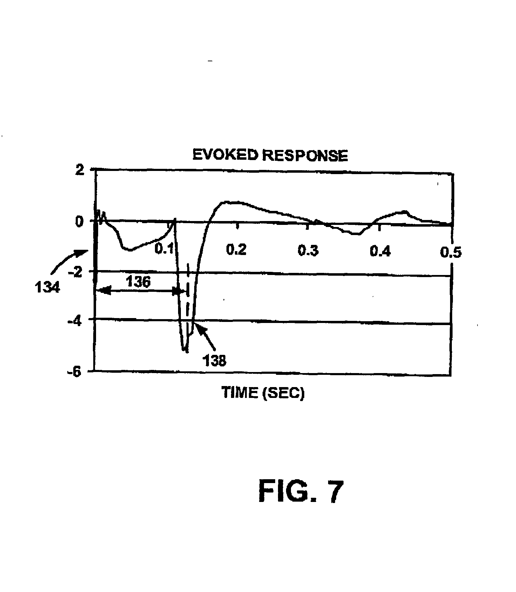

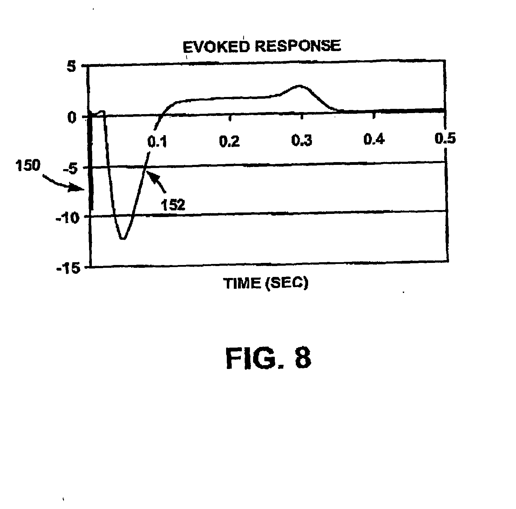

Continuous automatic capture verification is performed by sampling the signal received on the left and right ring electrodes 27 and 34, respectively, following delivery of a ventricular stimulation pulse (Vpace) on the left and right tip electrodes 26 and 32, respectively. Essentially, cross-ventricular sensing of the evoked R-wave is achieved. Using this cross-chamber sensing configuration, the R-wave depolarization in the left ventricle and the R-wave depolarization in the right ventricle will be sensed as a single complex as illustrated by the internal electromyogram (IEGM) recordings in **FIGS. 7** and **8**.

**FIG. 7** illustrates a situation of loss of capture, where a stimulation pulse 134 is followed by a time delay 136 followed by an intrinsic response 138 is observed. **FIG. 8** illustrates a situation of simultaneous capture in both the left and right ventricles. In this case, a stimulation pulse 150 is of sufficient energy to depolarize the cardiac tissue, resulting in a large evoked response 152 immediately following the stimulation pulse 150.

With further reference to **FIG. 2,** the IEGM signal received by ventricular sensing circuitry 84 is analyzed by the microcontroller 60 for determination of capture. A morphology detector 64 can be used to compare specific characteristics of the sampled signal with specific characteristics of an evoked response signal 152. Such characteristics include slope, event width, time to onset, and similar parameters. The overall signal morphology can also be compared to an evoked response signal template stored in memory 94. An appropriate method using signal morphology analysis for signal detection is generally described in U.S. Patent Number 4,817,605 to Sholder, which is incorporated herein by reference.

Yet a further aspect of the present invention is a programmable selection of cross-chamber, unipolar, or bipolar sensing. The embodiment illustrated in **FIG. 5** allows cross-chamber sensing of the R-wave produced in both ventricles using a unique cross-chamber electrode configuration. However, other cross-chamber sensing configurations are possible that will satisfy the object of the present invention in reliably sensing the evoked response. One such alternative sensing configuration is illustrated in **FIG. 6**, according to which, biventricular stimulation is performed in similar configuration as in **FIG. 5**, however biventricular bipolar sensing is performed in the left ventricle between the left atrial ($A_L$) ring electrode 27 (or a ventricular ring electrode (not shown)) and the left ventricular ($V_L$) tip electrode 26, and in the right ventricle between the right ventricular ($V_R$) ring electrode 34 and the right ventricular ($V_R$) tip electrode 32.

In practice, the medical practitioner will program the pacing polarity and direction by designating each electrode as a positive pole (or ground), a negative pole, or an inactive (unused) pole during stimulation. Likewise, the medical practitioner will program the sensing polarity and direction by designating each electrode as a positive pole, a negative pole, or inactive pole during sensing. As an illustrative example: If the left atrial ($A_L$) ring electrode 27 is programmed to be positive(+), the right ventricular ($V_R$) ring electrode 34 is programmed to be negative (-), the left ventricular ($V_L$) tip electrode 26, and the right ventricular ($V_R$) tip electrode 34 and the can case 40 (**FIG. 2**) are programmed to be inactive, then a cross-chamber sensing configuration from the right ventricle relative to the left ventricle has been selected.

Tables I and II below show additional illustrative examples of possible unipolar and bipolar combinations, where RV designates right ventricle, LV designates left ventricle, RA designates right atrium, and LA designates left atrium. The resulting polarities, sensing and stimulation pathway directions for various sensing configurations are shown based on the programmed electrode designation of: positive (+), negative (-), or inactive (0).

TABLE I

| Stimulation | Direction | RV tip 32 | RV ring 34 | LV tip 26 | LA ring 27 or LV ring 25 | Can 40 |
|---|---|---|---|---|---|---|
| Cross-chamber | Right to Left | - | 0 | + | 0 | 0 |
| Cross-chamber | Left to Right | + | 0 | - | 0 | 0 |

TABLE II

| Sensing | Direction | RV tip 32 | RV ring 34 | LV tip 26 | LA ring 27 or LV ring 25 | Can 40 |
|---|---|---|---|---|---|---|
| Cross-chamber | Right to Left | 0 | - | 0 | + | 0 |
| Cross-chamber | Left to Right | 0 | + | 0 | - | 0 |
| Bipolar both chambers | (conventional) | - | + | - | + | 0 |

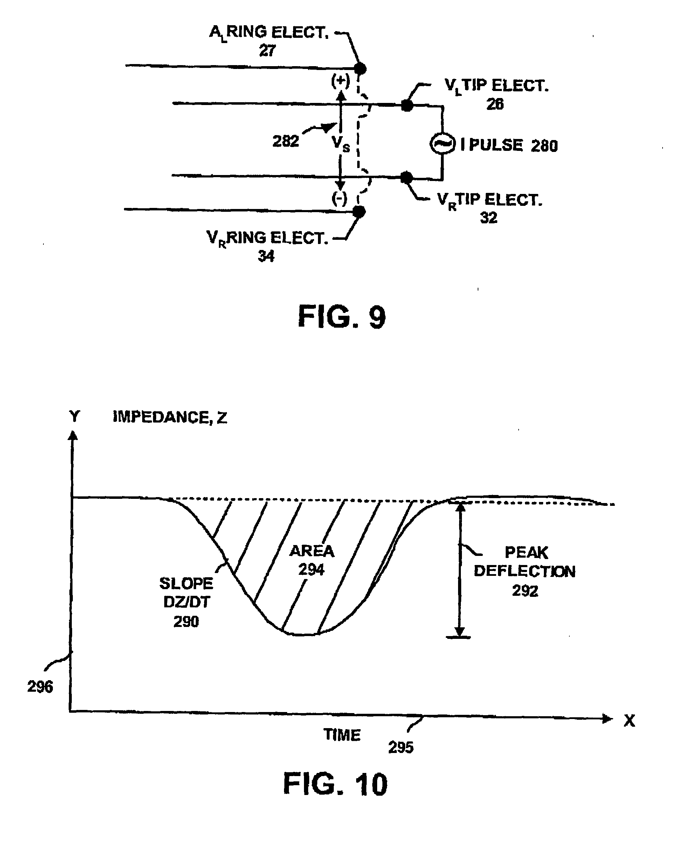

In another embodiment, impedance measurements can be made by an impedance measurement circuitry 112 (**FIG. 2**) for capture verification rather than evoked response detection from the IEGM. The use of the four electrode terminals (26, 27, 32, 34) on the coronary sinus lead 24 and the right ventricular lead 30, makes possible a highly sensitive cardiac impedance measurement. The equivalent circuit diagram of **FIG. 9** illustrates the measurement configuration. The application of an excitation current pulse 280 across the left ventricular ($V_L$) tip electrode 26 and the right ventricular ($V_R$) tip electrode 32, generates a voltage differential $V_S$ 282 that can be measured across the left atrial ($A_L$) ring electrode 27 and the right ventricular ($V_R$) ring electrode 34. The impedance can then be calculated by the microprocessor 60 according to the following equation:

$$Z=I/V,$$

where Z is the impedance associated with the myocardial tissue and blood volume residing between the left ventricular ($V_L$) tip electrode 26 and the right ventricular ($V_R$) tip electrode 32, I is the applied excitation current pulse 280, and $V_S$ is the measured voltage differential 282 that appears across the left atrial ($A_L$) ring electrode 27 and the right ventricular ($V_R$) ring electrode 34. Since the impedance measurement is made directly across the ventricles, it provides a direct measure of cardiac impedance with minimal influence of changes in thoracic impedance due to respiration. This more direct measure holds an advantage over impedance measurements that are performed between a lead electrode and the pacemaker can since, in those measurements, thoracic impedance may contribute to the measured signal to the same degree or an even greater degree than the cardiac impedance. Considerable signal processing is then needed to filter out the impedance signal associated with respiration.

During ventricular contraction, the cardiac impedance, Z, will change as a function of the changing blood volume in the heart chambers. Therefore, as blood is ejected from the right and left ventricles, the impedance will decrease and as the ventricles fill, the impedance will increase. Thus, a change in the measured impedance Z is a direct consequence of the actual mechanical contraction of the ventricles and serves as a reliable indication of capture.

**FIG. 10** is an illustration of the impedance signal change that may be measured during a ventricular contraction. The impedance Z is graphed along the Y-axis 296 versus time along the X-axis 295. The microprocessor 60 examines the impedance signal for specific characteristics that would indicate ventricular contraction has indeed occurred, such as the area of the curve 294, the peak slope dZ/dt 290, or the maximum peak deflection 292.

In an alternative embodiment of the present invention, stimulation is performed in one ventricle and capture sensing is performed in the other ventricle. More specifically, bipolar pacing is performed in the right ventricle between the right ventricular ($V_R$) tip electrode 32 and the right ventricular ($V_R$) ring electrode 34. Bipolar sensing is then performed between the left ventricular ($V_L$) tip electrode 26 and the left atrial ($A_L$) ring electrode 27.

When a supra-threshold pacing pulse is delivered, causing depolarization in the right ventricle, the depolarization wave will be conducted to the left ventricle via the conductive elements and the myocardial cells themselves. Thus, both ventricles are captured and this effect can be detected by sensing in the left ventricle using the non-polarized left ventricular ($V_L$) tip electrode 26 and left atrial ($A_L$) ring electrode 27. Such a mode of capture sensing is hereby referred to as "cross-tracking" in that sensing in one cardiac chamber is performed to verify capture due to pacing in another cardiac chamber.

Such cross-tracking can be performed to continuously monitor for capture during ventricular pacing or during periodic automatic threshold tests. While pacing pulse energy is progressively decreased in one ventricle, loss of capture is detected by sensing in the other ventricle. Specific algorithms for performing threshold tests are known by those reasonable skilled in the art and will not be described in detail here. For more detail, reference is made to U.S. Patent 5,766,229 to Bornzin, or U.S. provisional patent application No. 60/204,088, filed on May 15, 2000.

Thus a cardiac stimulating device has been described that provides reliable and efficient biventricular stimulation and capture sensing using cross-chamber electrode configurations thereby avoiding the difficulties associated with lead polarization in accurately detecting evoked responses for verifying capture.

## Claims

1. A cardiac stimulation system (10) for providing synchronous biventricular stimulation, comprising: a left ventricular lead (24) including a left ventricular electrode (26) that delivers stimulation pulses to a left ventricle; a right ventricular lead (30) including a right ventricular electrode (32) that delivers stimulation pulses to a right ventricle; a pulse generator (72) connected to the left ventricular lead (24) and the right ventricular lead (30) to capture the right and left ventricles; and an automatic capture detector (65) coupled to the pulse generator to verify capture of the left ventricle and the right ventricle, **characterised in that** the pulse generator (72) is adapted to perform biventricular stimulation with a cross-chamber configuration between the left ventricular electrode (26) and the right ventricular electrode (32), to synchronously capture the left and right ventricle.

2. A stimulation device as claimed in Claim 1, **characterised in that** the automatic capture detector programmably selects polarities for the right ventricular electrode and the left ventricular electrode to control an activation stimulation sequence.

3. A stimulation device as claimed in Claim 2, **characterised in that** the automatic capture detector performs automatic capture verification of the biventricular stimulation with a cross-chamber sensing configuration.

4. A stimulation device as claimed in Claim 3, **characterised by** an impedance measuring circuit that provides impedance measurements to the automatic capture detector for performing capture verification.

5. A stimulation device as claimed in any preceding Claim, **characterised in that** the right ventricular electrode and the left ventricular electrode are tip electrodes, the device further including a sensing electrode pair comprised of a right ventricular ring electrode and a left ventricular ring electrode.

6. A stimulation device as claimed in any preceding Claim, **characterised in that** the right ventricular lead is a bipolar lead that includes first and second right ventricular electrodes, in which the left ventricular lead is a bipolar lead that includes first and second left ventricular electrodes, and in which the automatic capture detector verifies capture in the right ventricle by delivering a stimulation pulse between the first and second right ventricular electrodes and by sensing a resulting voltage differential between the first and second left ventricular electrodes.

7. A stimulation device as claimed in any of Claims 1 to 5, **characterised in that** the right ventricular lead is a bipolar lead that includes first and second right ventricular electrodes, in which the left ventricular lead is a bipolar lead that includes first and second left ventricular electrodes, and in which the automatic capture detector verifies capture in the left ventricle by delivering a stimulation pulse between the first and second left ventricular electrodes and by sensing a resulting voltage differential between the first and second right ventricular electrodes.

8. A stimulation device as claimed in Claim 1, **characterised in that** the automatic capture detector confirms loss of capture by detecting a sequence of time delay and an intrinsic response immediately following a stimulation pulse.

9. A stimulation device as claimed in Claim 1, **characterised in that** the automatic capture detector confirms synchronous capture of the left and right ventricles by detecting an evoked response immediately following a stimulation pulse.

**Patentansprüche**

1. Ein Herzstimulationssystem (10) zur Lieferung einer synchronen biventrikulären Stimulation umfassend eine linke ventrikuläre Leitung (24) mit einer linken ventrikulären Elektrode (26), die Stimulationspulse an ein linkes Ventrikel liefert; eine rechte ventrikuläre Leitung (30) mit einer rechten ventrikulären Elektrode (32), die Stimulationspulse an ein rechtes Ventrikel liefert; einen Pulsgenerator (72), der mit der linken ventrikulären Leitung (24) und der rechten ventrikulären Leitung (30) verbunden ist, um das rechte und das linke Ventrikel zu erfassen; und einen automatischen Capture-Detektor (65), der mit dem Pulsgenerator gekoppelt ist, um eine Erfassung des linken Ventrikels und des rechten Ventrikels zu verifizieren, **dadurch gekennzeichnet, dass** der Pulsgenerator (72) geeignet ist, eine biventrikuläre Stimulation mit einer Kreuzkammerkonfiguration zwischen der linken ventrikulären Elektrode (26) und der rechten ventrikulären Elektrode (32) durchzuführen, um das linke und das rechte Ventrikel synchron zu erfassen.

2. Eine Stimulationsvorrichtung nach Anspruch 1, **dadurch gekennzeichnet, dass** der automatische Capture-Detektor die Polaritäten der rechten ventrikulären Elektrode und der linken ventrikulären Elektrode programmierbar auswählt, um eine Aktivierung der Stimulationssequenz zu steuern.

3. Eine Stimulationsvorrichtung nach Anspruch 2, **dadurch gekennzeichnet, dass** der automatische Capture-Detektor eine automatische Capture-Verifizierung der biventrikulären Stimulation mit einer Kreuzkammer-Abtastkonfiguration durchführt.

4. Eine Stimulationsvorrichtung nach Anspruch 3, **gekennzeichnet durch** eine Impedanzmeßschaltung, die Impedanzmessungen an dem automatischen Capture-Detektor zur Durchführung der Capture-Verifikation liefert.

5. Eine Stimulationsvorrichtung nach einem der vorhergehenden Ansprüche, **dadurch gekennzeichnet, dass** die rechte ventrikuläre Elektrode und die linke ventrikuläre Elektrode Spitzenelektroden sind, wobei die Vorrichtung femer ein Sensorelektrodenpaar umfasst, das aus einer rechten ventrikulären Ringelektrode und einer linken ventrikulären Ringelektrode besteht.

6. Eine Stimulationvorrichtung nach einem der vorhergehenden Ansprüche, **dadurch gekennzeichnet, dass** die rechte ventrikuläre Leitung eine bipolare Leitung ist, die erste und zweite, rechte ventrikuläre Elektroden umfasst, dass die linke ventrikuläre Leitung eine bipolare Leitung ist, die erste und zweite, linke ventrikuläre Elektroden umfasst, und dass der automatische Capture-Detektor die Erfassung in dem rechten Ventrikel durch Abgeben eines Stimulationspulses zwischen der ersten und der zweite, rechten ventrikulären Elektrode und durch Abtasten einer resultierenden Spannungsdifferenz zwischen der ersten und der zweiten, linken ventrikulären Elektrode verifiziert.

7. Eine Stimulationsvorrichtung nach einem der Ansprüche 1 bis 5, **dadurch gekennzeichnet, dass** die rechte ventrikuläre Leitung eine bipolare Leitung ist, die erste und zweite, rechte ventrikuläre Elektroden umfasst, dass die linke ventrikuläre Leitung eine bipolare Leitung ist, die erste und zweite, linke ventrikuläre Elektroden umfasst, und dass der automatische Capture-Detektor die Erfassung in dem linken Ventrikel durch Abgeben eines Stimulationspulses zwischen der ersten und der zweiten, linken ventikulären Elektrode und durch Abtasten einer resultierenden Spannungsdifferenz zwischen der ersten und der zweiten, rechten ventrikulären Elektrode verifiziert.

8. Eine Stimulationsvorrichtung nach Anspruch 1, **dadurch gekennzeichnet, dass** der automatische Capture-Detektor den Verlust der Erfassung **dadurch** bestätigt, dass eine Sequenz einer Zeitverzögerung und eine intrinsische Antwort unmittelbar auf einen Stimulationspuls folgend detektiert wird.

9. Eine Stimulationsvorrichtung nach Anspruch 1, **dadurch gekennzeichnet, dass** der automatische Capture-Detektor die synchrone Erfassung des linken und des rechten Ventrikels **dadurch** bestätigt, dass eine hervorgerufene Antwort unmittelbar folgend auf einen Stimulationspuls detektiert wird.

**Revendications**

1. Système de stimulation cardiaque (10) pour fournir une stimulation biventriculaire synchrone, comprenant : une sonde de ventricule gauche (24) comprenant une électrode de ventricule gauche (26) qui délivre des impulsions de stimulation à un ventricule gauche; une sonde de ventricule droit (30) comprenant une électrode de ventricule droit (32) qui délivre des impulsions de stimulation à un ventricule droit; un générateur d'impulsions (72) connecté à la sonde de ventricule gauche (24) et à la sonde de ventricule droit (30) pour capturer les ventricules droit et gauche; et un détecteur de capture automatique (65) couplé au générateur d'impulsions pour vérifier la capture du ventricule gauche et du ventricule droit, **caractérisé en ce que** le générateur d'impulsions (72) est adapté pour effectuer une stimulation biventriculaire avec une configuration intercavités entre l'électrode de ventricule gauche (26) et l'électrode de ventricule droit (32), pour capturer de manière synchrone les ventricules gauche et droit.

2. Dispositif de stimulation selon la revendication 1, **caractérisé en ce que** le détecteur de capture automatique sélectionne de manière programmable des polarités pour l'électrode de ventricule droit et l'électrode de ventricule gauche pour commander une séquence de stimulation d'activation.

3. Dispositif de stimulation selon la revendication 2, **caractérisé en ce que** le détecteur de capture automatique effectue une vérification de la capture automatique de la stimulation biventriculaire avec une configuration de détection intercavités.

4. Dispositif de stimulation selon la revendication 3, **caractérisé par** un circuit de mesure d'impédance qui fournit des mesures d'impédance au détecteur de capture automatique pour effectuer la vérification de la capture.

5. Dispositif de stimulation selon l'une quelconque des revendications précédentes, **caractérisé en ce que** l'électrode de ventricule droit et l'électrode de ventricule gauche sont des électrodes en pointe, le dispositif comprenant en outre une paire d'électrodes de détection composée d'une électrode en anneau pour le ventricule droit et d'une électrode en anneau pour le ventricule gauche.

6. Dispositif de stimulation selon l'une quelconque des revendications précédentes, **caractérisé en ce que** la sonde de ventricule droit est une sonde bipolaire qui comprend une première et une seconde électrodes de ventricule droit, la sonde de ventricule gauche est une sonde bipolaire qui comprend une première et une seconde électrodes de ventricule gauche, et le détecteur de capture automatique vérifie la capture dans le ventricule droit en délivrant une impulsion de stimulation entre les première et seconde électrodes de ventricule droit et en détectant une différence de tension résultante entre la première et la seconde électrodes de ventricule gauche.

7. Dispositif de stimulation selon l'une quelconque des revendications 1 à 5, **caractérisé en ce que** la sonde de ventricule droit est une sonde bipolaire qui comprend une première et une seconde électrodes de ventricule droit, la sonde de ventricule gauche est une sonde bipolaire qui comprend une première et une seconde électrodes de ventricule gauche, et le détecteur de capture automatique vérifie la capture dans le ventricule gauche en délivrant une impulsion de stimulation entre les première et seconde électrodes de ventricule gauche et en détectant une différence de tension résultante entre la première et la seconde électrodes de ventricule droit.

8. Dispositif de stimulation selon la revendication 1, **caractérisé en ce que** le détecteur de capture automatique confirme la perte de capture par la détection d'une séquence comportant un retard temporel et une réponse intrinsèque immédiatement après une impulsion de stimulation.

9. Dispositif de stimulation selon la revendication 1, **caractérisé en ce que** le détecteur de capture automatique confirme la capture synchrone des ventricules gauche et droit par la détection d'une réponse provoquée immédiatement après une impulsion de stimulation.

**FIG. 1**

**FIG. 2**

**FIG. 3**

EP 1 155 711 B1

FIG. 4

**FIG. 5**

**FIG. 6**

19

FIG. 7

**FIG. 8**

FIG. 9

FIG. 10